# EUROPEAN PATENT APPLICATION

(11) **EP 1 918 713 A1**
(43) Date of publication of application: **07.05.2008**
(21) Application number: 06123235.1
(22) Date of filing: 31.10.2006
(51) Int. Cl.: G01N 33/68

(54) **Analysis of proteolytic processing by mass spectrometry**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The present invention relates to the simultaneous analysis of samples for determining differential proteolytic processing based on isotopic labelling of N-terminal peptides, wherein the isotopic labelling is achieved by incorporation of ¹⁸O during enzymatic proteolysis.

## Description

### FIELD OF THE INVENTION

The present invention relates to tools and methods for determining proteolytic processing simultaneously in different samples using MS analysis.

### BACKGROUND OF THE INVENTION

Proteases were initially characterised as non-specific degradative enzymes that are associated with protein catabolism. However, it is becoming increasingly recognised that proteolysis is an important mechanism for achieving precise cellular control of biological processes in all living organisms, through the highly specific cleavage of certain proteins [Barrett (1998) in "Handbook of Proteolytic Enzymes" Academic Press, London]. This highly specific and limited substrate cleavage is termed proteolytic processing. Proteases, through their ability to catalyse irreversible hydrolytic reactions, regulate the fate and activity of many proteins by controlling appropriate intra- or extracellular localisation, by shedding from cell surfaces, by activation or inactivation of proteases and other enzymes, cytokines, hormones or growth factors, by conversion of receptor agonists to antagonists and by exposure of cryptic neoproteins (i.e. the proteolytic cleavage product is a functional protein with a role that is distinct from the parent protein). Hence, proteases initiate, modulate and terminate a wide range of important cellular functions by processing bioactive molecules, and thereby directly control essential biological processes, such as DNA replication, cell-cycle progression, cell proliferation, differentiation and migration, morphogenesis and tissue remodelling, neuronal outgrowth, haemostasis, wound healing, immunity, angiogenesis and apoptosis (reviewed e.g. in Stemlicht et al. (2001), Ann. Rev. Cell. Dev. Biol. 17, 463-516).

Considering the functional relevance of proteases for all living processes, including cell death, it is not difficult to understand that a deficiency, or a misdirected temporal and spatial activity, of these enzymes underlies several pathological conditions such as cancer, arthritis, neurodegenerative and cardiovascular diseases. Moreover, many infectious microorganisms, viruses and parasites use proteases as virulence factors, and animal venom commonly contains proteases to effect tissue destruction or to evade host responses. Accordingly, many proteases or their substrates are an important focus of attention for the pharmaceutical industry as potential drug targets.

Owing to the expanding roles for proteolytic enzymes, there has been an increasing interest in the identification and functional characterisation of the many proteases that are present in various organisms, from bacteria to man. The near completion of several large-scale genome-sequencing projects has provided new opportunities to appreciate the complexity ofprotease systems. The human genome contains more than 500 genes that encode proteases or protease-like molecules.

Despite the increased knowledge on the proteases, the substrates and *in vivo* roles for newly identified proteases are unknown and, even for proteases that have been well characterised, their biological functions are often not fully understood. New techniques are urgently required to identify the protease repertoire that is expressed and active in a cell, tissue or organism, as well as to identify all the natural substrates of each protease.

Evidence for the increasing complexity and importance of the proteolytic systems that function in all organisms, and the ability to analyse systems in their entirety on genome- and proteome-wide scales, necessitates the introduction of new terms to clarify emerging concepts in this field. So, 'degradomics' was first coined to define the substrate repertoire of a protease on a proteome-wide scale by McQuibban et al. (2000), Science 289, 1202-1206. In addition, the term is also used for the complete set of proteases that are expressed at a specific moment or circumstance by a cell, tissue or organism. The field of degradomics will be built using emerging, and new, genomic and proteomic technologies to investigate and define both types of protease degradome.

Identifying the substrate degradomes of individual proteases will facilitate our understanding of their physiological and pathological roles and thereby point to new diagnostic biomarkers, as well as to novel drug targets. This information, in conjunction with knowledge of the protease degradome of a cell, will increase our understanding of the biological roles of proteases in the cellular context with respect to cell function and pathology. Similar information on a tissue-wide scale should prove useful in the molecular diagnosis of disease, with the calibration of protease levels to disease severity or tumour grade enabling more accurate prognostic predictions to be made for patients.

Functional degradomics has two branches: the first is based on activity profiling of individual proteases, and the second involves determination of the cleavage of target substrates. So, instead of defining individual contributions by specific proteases, this latter aim considers the protease degradome as a system that leads to substrate cleavage. The field of degradomics promises to uncover new proteases and physiological substrates, and to identify new and known regulatory pathways that are controlled by proteolytic processing. The regulation of these pathways might be disrupted in disease states, or host proteases might be used by micro-organisms for infection, and could therefore be therapeutically targeted. Different proteomic methods are described to study proteolytic processing.

Hancock et al. (W02006044666) isolate the low Mr peptide fraction of a sample and identify the proteins therein. No information is obtained on the processed proteins themselves, which remain in the high Mr protein fraction.

McDonald et al. ((2005) Nat. Methods 2, 955-957), describe a method wherein N-terminal peptides from a protein mixture are isolated and identified by MS. This method succeeds in identifying a novel cleavage site in a liver protein. In this method only one sample is studied and all peptides, also those from non-processed proteins need to be verified by MS and sequence determination to reveal eventual novel proteolytic processing events.

Overall *et al.* on the other hand use a method wherein two samples are assayed simultaneously, using ICAT (Isotope-Coded Affinity Tag) labels [Overall and Dean (2006) Cancer Metastatis 25, 69-75]. In this method the thiol group of cysteine is modified with an affinity tagged label, the sample is digested with trypsin, and the labelled peptides are isolated. In this way proteins are detected which have different expression levels due to the degradation of aberrant processed proteins or due to increased shedding. This method however gives no information on the cleavage site in these proteins.

Fisher et al (US20060134723) describe methods to study protein maturation and processing in different samples using isotopic labelling and by selecting N-terminal or C-terminal peptides.

There remains a need for analysis methods which allow an efficient analysis of proteolytic processing in a sample.

### SUMMARY OF THE INVENTION

The present invention provides methods for comparing the proteolytic processing between two protein samples which are based on the selective labelling and isolation of N-terminal peptides. The selective labelling and isolation of N-terminal peptides is achieved by a combination of protein cleavage with H₂¹⁸O isotopic labelling followed by isolation of N-terminal peptides for further analysis. This method has the advantage that the number of handling steps is reduced in comparison with prior art methods wherein isotopic labelling and protein cleavage are performed in two separate steps. In addition, expensive isotopic labelling compounds are substituted by less expensive H₂¹⁸O. By combining the protein cleavage and the labelling with H₂¹⁸O every peptide that is cleaved will automatically be labelled which increases the efficiency of the labelling. The protease mediated ¹⁸O incorporation has the additional advantage that it is specific for C-termini and does not interfere with the functional carboxylgroup of internal amino acids Asp and Glu, where present.

The present invention further provides multiplex double labelling methods wherein protease-mediated O¹⁸ incorporation and amine specific isobaric labelling are combined. In these methods, the isobaric labelling is combined with a protein modification step, which normally are performed as two separate steps.

Particular and preferred aspects of the invention are set out in the accompanying independent and dependent claims. Features from the dependent claims may be combined with features of the independent claims and with features of other dependent claims as appropriate and not merely as explicitly set out in the claims.

A first aspect of the present invention provides *in vitro* methods for comparing proteolytic processing between two or more different protein samples. The methods according to this aspect of the invention comprise the steps of (a) modifying the amine of the N-terminus and of Lysine residues of the proteins in said samples, (b) cleaving the modified proteins into peptides and simultaneously labelling each of the samples with either O or ¹⁸O by protease-induced incorporation of O or ¹⁸O, (c) isolating from the obtained peptides the N-terminal peptides and (e) subjecting the N-terminal peptides to MS. The methods according to this aspect of the invention further comprise the step of pooling (d) the labelled samples obtained in step (b) or the isolated N-terminal peptides obtained in step (c). In a next step (f), the relevant peptide fractions are selected based on the MS analysis in step (e), and these relevant peptide fractions are optionally further analysed (g) to identify the peptides therein.

According to one embodiment, the methods comprise, after step (c), the step of subjecting the isolated N-terminal peptides to a peptide separation step.

In particular embodiments of the methods of the present invention the modification in step (a) is performed for each sample with different isobaric labelling reagents comprising an amine reactive group. In these embodiments, the modification step is a differential labelling step, whereby a different label is incorporated in each sample.

In further particular embodiments of the methods of the invention, the cleavage in step (b) is performed with trypsin.

In particular embodiments of the methods of the invention, the isolation of N-terminal peptides is performed by covalently linking an affinity tag to the N-terminus of the internal and C-terminal peptides, and removing the internal and C-terminal peptides from the samples by affinity chromatography.

In particular embodiments of these methods, step (g) comprises analysing the identified protein samples on MS/MS.

In particular embodiments of the methods according to this aspect of the invention, when two samples are used, the selection step in step (f) consists of identifying those peaks for which the ratio between the peaks of the isotopically labelled peptides is below 0,5 or above 1,5, more particularly below 0,1 or above 10.

In to particular embodiments the methods of the invention are applied to protein samples of which one or more are samples from tumour patients.

A second aspect of the present invention relates to the use of the above-described methods for determining proteolytic cleavage sites in the characterisation of enzymes, proteins, cleavage conditions, disease states, etc..

A further aspect of the present invention relates to the use of the above described methods for determining downstream effects of proteolytic processing.

Yet a further aspect of the present invention provides tools for performing the methods of the present invention, more particularly kits of reagents for the differential labelling of samples, which are characterized in that they comprise a set of two or more isobaric labelling reagents and H₂¹⁸O.

In one embodiment, the kits further comprise means for isolating polypeptides with a free N-terminus.

In yet a further aspect of the present invention, devices are provided which are particularly suited for carrying out the methods described herein. The devices (100') provided in the present invention for the simultaneous analysis of two protein samples using isotopic labelling typically comprise two sample sources (101), a protein modification unit (103') with a source of modifying reagent (104'), a labelling and protein cleavage unit (105) for protease mediated labelling with either ¹⁶O or ¹⁸O and corresponding label sources (107), a N-terminal peptide isolation unit (106), a separation unit (108), a mass spectrometer unit (109) and a data analysis unit (110).

Yet a further aspect of the present invention provides devices (100) for multiplex analysis of two or more protein samples using double labelling comprising at least two sample sources (101), a labelling unit (103) with at least two sources of labelling reagent (104), a labelling and protein cleavage unit (105) for protease mediated labelling with either ¹⁶O or ¹⁸O and corresponding label sources (107), an N-terminal peptide isolation unit (106) a separation unit (108), a mass spectrometer unit (109) and a circuitry control and data analysis unit (110).

Particular embodiments of the devices described above further comprise a sample preparation unit (102).

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE DRAWINGS

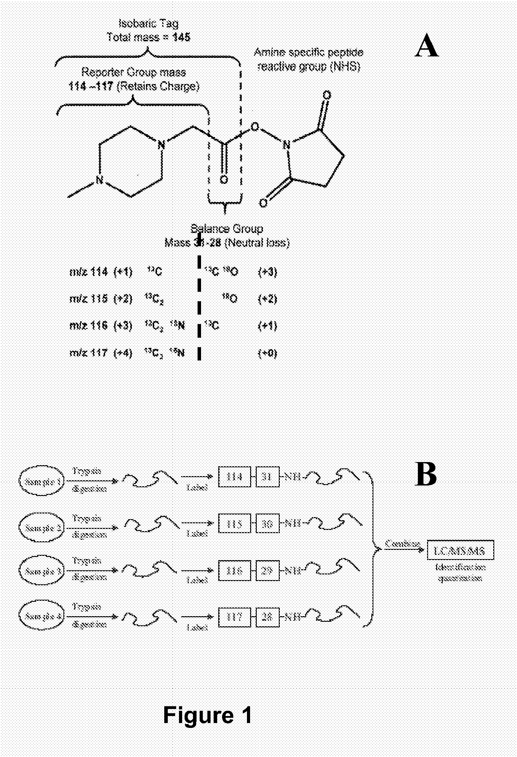

Fig. 1 shows exemplary structures of iTRAQ reagents (A) and peptides labelled therewith (B). The detailed structure of an isobaric labelling reagent according to an embodiment of the present invention consisting of a reporter group with a mass ranging from 114 to 117 Da, a balance group with a mass ranging from 31 to 28 Da and an amine-specific peptide reactive group (NHS).

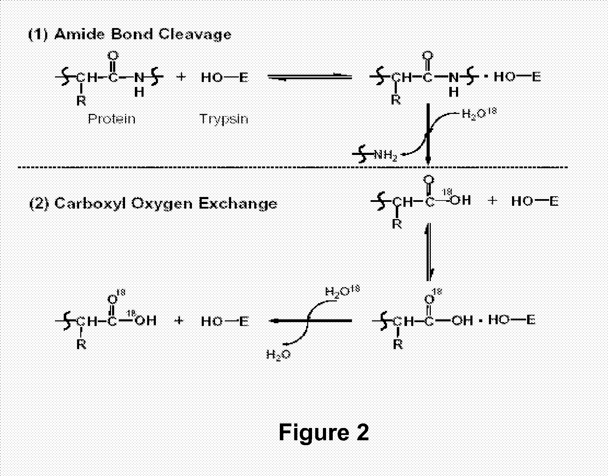

Fig. 2 illustrates trypsin-mediated incorporation of ¹⁸O into both carboxyl oxygen atoms of the C-terminal amino acid of a cleaved peptide (E: enzyme).

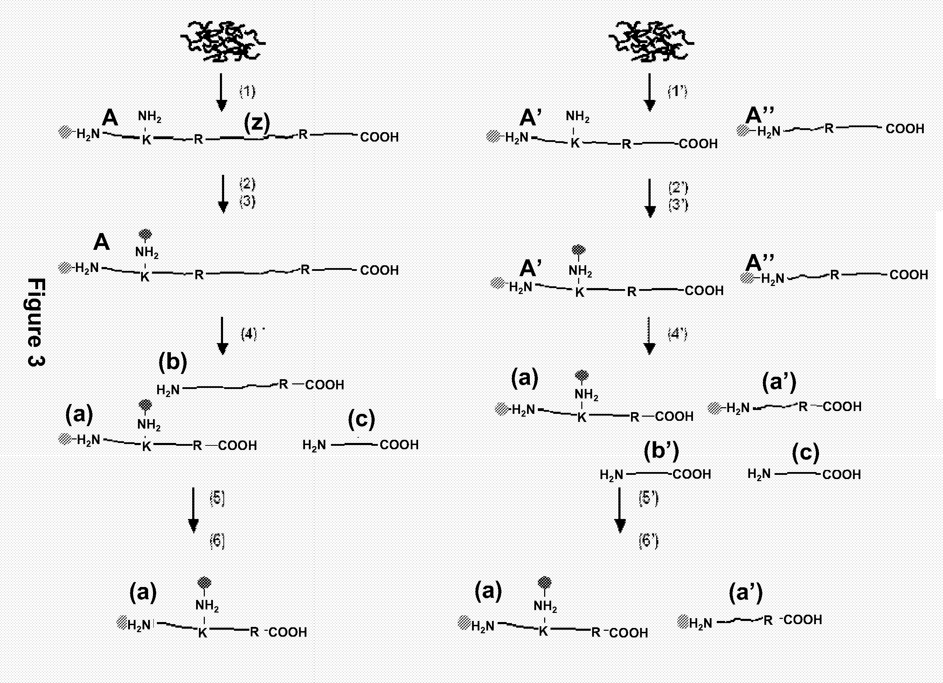

Fig. 3 demonstrates the identification of a differently processed protein in two samples in accordance with particular embodiments of the present invention. (1): protein denaturation; (2): modification of cysteines; (3): modification of primary amines; (4): enzymatic digest; (5): isolation of N-terminal peptides; The left panel shows an *in vivo* unprocessed protein 'A'. After cleavage the protein 'A' is cleaved into an N-terminal peptide (a) an internal peptide (b) and a C-terminal peptide (c). In the right panel protein A in the sample is *in vivo* processed at amino acid (z) into two fragments A' and A". Upon digestion A' is cleaved into the N-terminal (a) and an internal/c-terminal peptide (b'). A" is cleaved into an N-terminal peptide (a') and the c-terminal peptide (c). Selection ofN -terminal peptides isolates peptide (a) in the left panel and peptides (a) and (a') in the right panel.

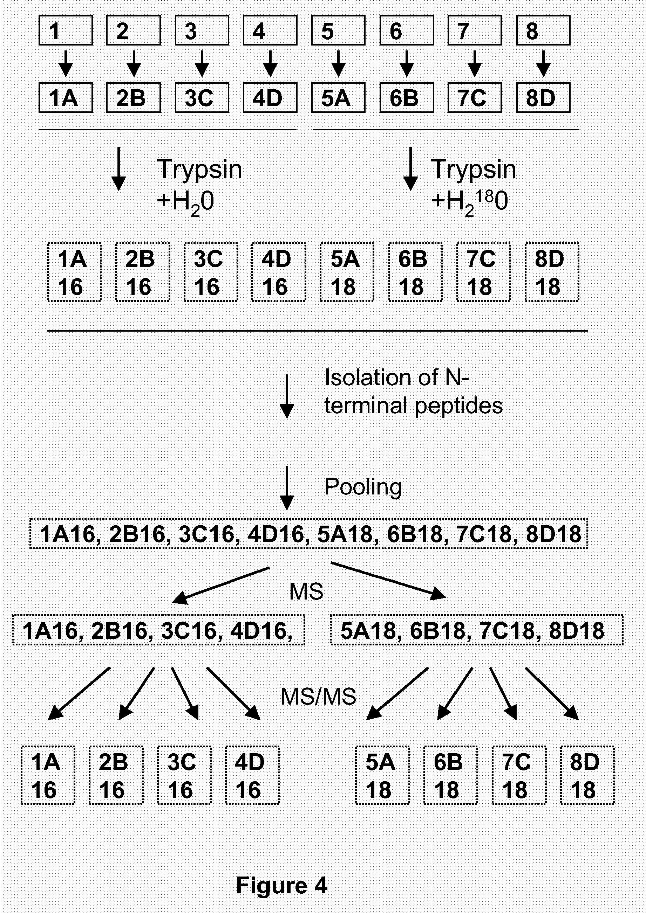

Fig. 4 illustrates the simultaneous analysis of multiple samples using double labelling with ¹⁸O isotopic labelling and amine specific isobaric labelling in accordance with particular embodiments of the present invention. 1-8: samples; A-D isobaric labels, 16 and 18 are isotopic labels.

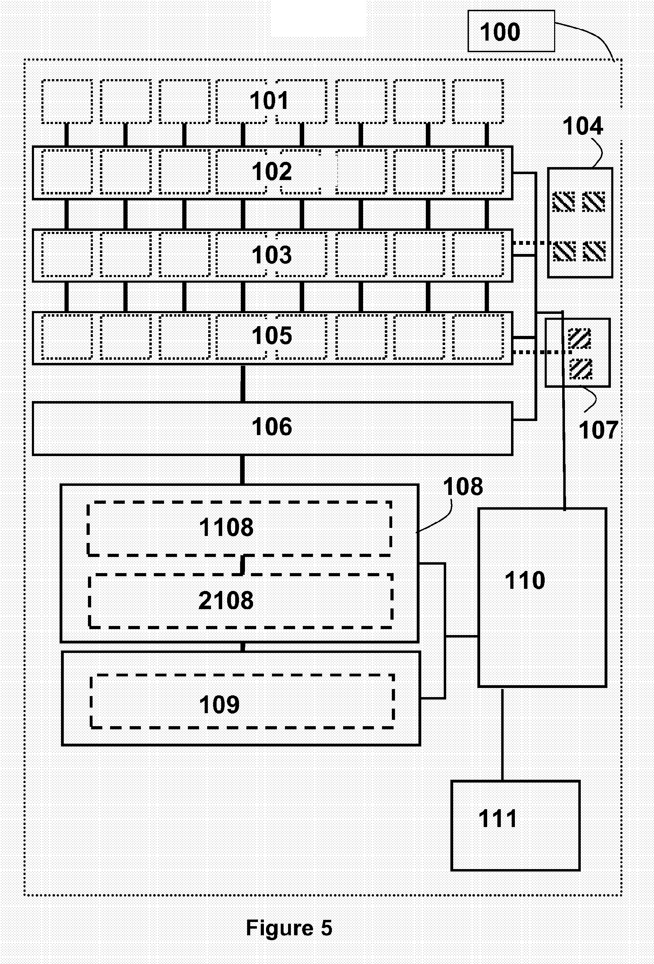

Fig. 5 shows in accordance with a particular embodiment of the present invention a device (100) for multiplex analysis of 8 protein samples using double labelling, comprising eight sample sources (101), a sample preparation unit (102), a (first) labelling unit (103) with corresponding first label sources (104), a cleavage and (second) labelling unit (105), with corresponding second label (H₂¹⁶O H₂¹⁸O) sources (107), an N-terminal peptide isolation unit (106), a separation unit (108) comprising two consecutively linked separation systems (1108) and (2108), a mass spectrometer unit (109) and a control circuitry and data analysis unit (110) coupled to a read out system (111).

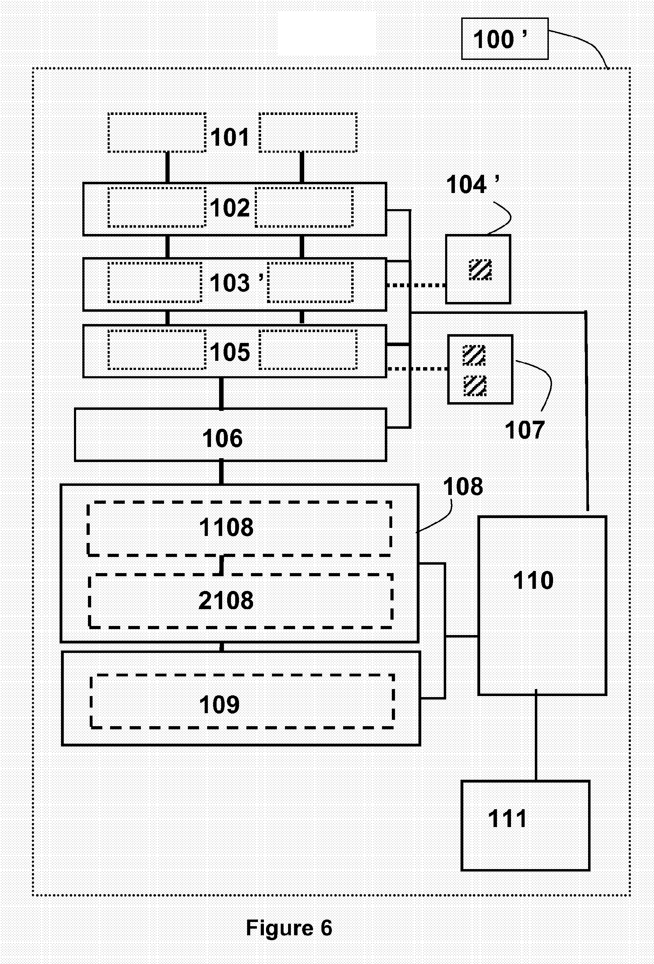

Fig. 6 shows in accordance with a particular embodiment of the present invention a device (100') for analysis of 2 protein samples using isotopic labelling, comprising two sample sources (101), a sample preparation unit (102), a protein modification unit (103') with a source of modifying reagent (104'), a cleavage and labelling unit (105) with corresponding sources of labelling reagents (H₂¹⁶O and H₂¹⁸O) (107), an N-terminal peptide isolation unit (106), a separation unit (108) comprising two consecutively linked separation systems (1108) and (2108), a mass spectrometer unit (109), a control circuitry and data analysis unit (110) coupled to a read out system (111).

### DETAILED DESCRIPTION OF THE EMBODIMENTS

In the different Figures, the same reference signs refer to the same or analogous elements.

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specified, these definitions should not be construed to have a scope less than understood by a person of ordinary skill in the art.

The term "polypeptide" or "protein", as used herein, refers to a plurality of natural or modified amino acids connected via a peptide bond. The length of a polypeptide can vary from 2 to several thousand amino acids (the term thus also includes what is generally referred to as oligopeptides). Included within this scope are polypeptides comprising one or more amino acids which are modified by *in vivo* posttranslational modifications such as glycosylation, phosphorylation, etc. and/or comprising one or more amino acids which have been modified *in vitro* with protein modifying agents (e.g. alkylating and acetylating agents).

The term "polypeptide fragment" or "peptide" as used herein is used to refer to the amino acid sequence obtained after enzymatic cleavage of a protein or polypeptide. A polypeptide fragment or peptide is not limited in size or nature.

The terms "internal", "N-terminal" and "C-terminal" when referring to a peptide are used herein to refer to the corresponding location of a peptide in a protein or polypeptide. For example, in a tryptic cleavage of protein NH₂-X₁-K-X₂-R-X₃-K-X₄-COOH (wherein X₁, X₂, X₃ and X₄ are peptide sequences of undetermined length without Lysine (K) or Arginine (R), ), the N-terminal peptide is NH₂-X₁-K-COOH, the internal peptides are NH₂-X₂-R-COOH and NH₂-X₃-K-COOH and the C-terminal peptide is NH₂-X₄-COOH.

The term "degradome", when used herein in the context of the degradome of a cell refers to, the complete set of proteases that are expressed at a specific moment or circumstance by a cell, tissue or organism. The same term, when used herein, in the context of a protease can refer to the substrate repertoire of that protease in a cell, tissue or organism.

The term "protein cleavage" as used herein relates to the hydrolysis of a peptide bond between two amino acids in a polypeptide. In the methods of the present invention, protein cleavage is performed enzymatically. In the context of physiologic processes terms such as "enzymatic hydrolysis", "proteolytic processing", and "protein maturation" are also used.

The term "fragmentation" as used herein refers to the breaking of one or more chemical bonds and subsequent release of one or more parts of a molecule as obtained e.g. by collision-induced dissociation (CID) in Mass spectrometry (MS). In certain embodiments the bond is a peptide bond, but it is not limited thereto.

The term "label" as used herein refers to a compound or molecule, which can be covalently linked to or incorporated in a peptide or polypeptide and which, based on its particular properties is detectable on a mass spectrometer. Labels include composite chemical molecules which can be covalently bound to a peptide or polypeptide through a protein/peptide reactive group, present in the labelling reagent. Labels also include single atoms (e.g. an isotope), which are incorporated into the peptide or polypeptide of interest by way of a chemical and/or enzymatic reaction. Though the term label is used in a general sense, a distinction can be made between the label molecule as bound to a protein or peptide and the labelling reagent (more specifically referring to the compounds comprising the label prior to the binding with the peptide or protein, comprising a reactive group for binding on a protein or peptide). The present invention envisages the use of different types of labels, such as isotopic and isobaric labels defined below. The term 'set of labels' as used herein with regard to isotopic or isobaric labels (or labelling reagents), refers to two or more different labels or labelling reagents which can be used simultaneously in one experiment to label different samples, i.e. which have the same chemical structure but can be differentiated based on mass in MS or MS/MS.

The term "isotopic label(s)" as used herein refers to a set of molecules with essentially the same structure and behaving in the same way in electrophoresis and chromatography, but differing in one or more atoms to generate a difference in mass, and which can be used as (part of) a label. The difference in mass between different isotopic label components is ensured by replacement of an atom with an isotope of the same atom. Identical peptides each labelled with a label comprising a label component with the same or essentially the same chemical formula, but differing in mass based on the presence of different isotopes of the same atoms (either in number or type) can be distinguished from each other in MS..

The term "isotopic O label" as used herein refers to a label comprising one or more different ¹⁸O atoms. The combined use of the incorporation of one or more ¹⁸O in one sample or set of samples, with the incorporation of one or more oxygen or ¹⁶O (most often referred to as O herein) in another sample or an alternative set of samples, results in the isotopic labelling of these samples. The isotopic O label is incorporated as an alternative to O in the C-terminus of a peptide during enzymatic proteolysis resulting in a difference in mass on MS between those peptides comprising a C-terminal O and those comprising a C-terminal ¹⁸O. Accordingly, identical peptides labelled differentially with an isotopic O label can be differentiated as such on MS based on difference in mass.

The term "isobaric labels" as used herein refers to a set of labels having the same structure, and the same mass, which upon fragmentation release a particular fragment with the same structure for all isobaric labels of that set, which differs in mass between the individual isobaric labels in that set, due to a differential distribution of isotopes within the isobaric labels. Isobaric labels typically comprise a reporter group (RG), which is a relatively small fragment and a balance group (BG). The "combined mass" of a set of isobaric labels refers to the total mass of the reporter group and the balance group for that set of isobaric labels.

The term "reporter group (RG)" refers to the part of isobaric labels which generates a strong signature ion upon Collision Induced Dissociation (CID). The typical fragments generated upon release of the reporter group are used to quantitate the corresponding isobarically-labelled polypeptide. Typically the fragment ions appear in the low-mass region of an MS spectrum, where other fragment ions are not generally found.

The term "balance group (BG)" as used herein refers to the part of isobaric labels, which contains a certain compensating number of isotopes so as to ensure that the combined mass of the reporter group and balance group is constant for the different isobaric labels of one set. The balance group may or may not be released from the label upon CID.

The term "protein/peptide reactive group" (PRG) as used herein refers to a chemical function on a compound that is capable of reacting with a functional group on an amino acid of a protein or peptide resulting in the binding (non-covalent or covalent) of such compound to the amino acid. Typically labelling reagents comprise a PRG whereby upon interaction of the PRG with a functional group on the peptide or protein, the label is bound thereto.

The term "functional group" as used herein refers to a chemical function on an amino acid which can be used for binding (generally, covalent binding) to a chemical compound. Functional groups can be present on the side chain of an amino acid or on the N-terminus or C-terminus of a polypeptide or peptide. The term encompasses both functional groups which are naturally present on a peptide or polypeptide and those introduced via e.g. a chemical reaction using protein-modifying agents.

The methods of the present invention allow the accurate comparison of proteolytic processing events in two or more samples at the mass spectrometry level, whereby a minimal number of peptides generated in these samples needs to be analysed without loosing valuable data. The combination of protein cleavage and selection ofN-terminal peptides restricts the analysis to a pooled sample wherein each polypeptide of the original samples is represented by one N-terminal peptide.

The invention is directed to methods for detecting differences in proteolysis between two or more samples. Generally the sample will be of mammalian origin. However, other organisms can be used to study proteolytic processing by e.g. inactivating or overexpressing genes of proteases and protease inhibitors in model organisms such as zebrafish, *Drosophila, C. elegans, S. p*ombe or *S. cerevisiae.* In particular embodiments the sample is a tissue sample or cultivated cells from a tissue sample. In other embodiments, the sample is a bodily fluid such as blood (e.g., plasma or serum), saliva, urine, nipple aspirate, ductal lavage, sweat or perspiration, tumor exudates, joint fluid (e.g. synovial fluid), inflammation fluid, tears, semen and vaginal secretions.

In particular embodiments, samples are samples of mammalian origin, which have been in contact with poisons from snakes, scorpions and the like.

In further particular embodiments, samples are samples of mammalian origin wherein a gene is transfected coding for an active protease, an inactivated protease, an active protease inhibitor or an inactivated protease inhibitor.

In what follows, reference will generally be made to a "sample" thereby referring to either a non-purified or purified protein comprising material of a particular origin. Such a sample can comprise one or more proteins according to the present invention. To simplify the description, reference will generally be made to "a protein" in a sample. This is not intended to limit the methods of the present invention to the analysis of one-protein samples. To the contrary, the invention envisages the use of the methods of the present invention for the analysis of complex samples, whereby the presence and proteolytic processing of different proteins in each sample can be compared within one analysis.

The methods and tools of the present invention relate to the analysis of protein samples. As indicated above, the term "sample" as used herein is not intended to necessarily include or exclude any processing steps prior to the performing of the methods of the invention. The samples can be rough unprocessed samples, extracted protein fractions, purified protein fractions etc...

According to one embodiment the protein samples are pre-processed by immunodepletion of abundant proteins.

The preparation of samples differs depending on the organism, tissue or organ investigated, but standard procedures are usually available and known to the expert. With respect to mammalian and human protein samples it covers the isolation of cultured cells, laser micro-dissected cells, body tissue, body fluids, or other relevant samples of interest. With respect to the fractionation of proteins in a sample, cell lysis is the first step in cell fractionation and protein purification. Many techniques are available for the disruption of cells, including physical, enzymatic and detergent-based methods. Historically, physical lysis has been the method of choice for cell disruption; (homogenisation, osmotic lysis, ultrasound cell disruption) however, it often requires expensive, cumbersome equipment and involves protocols that are sometimes difficult to repeat due to variability in the apparatus (such as loose-fitting compared with tight-fitting homogenisation pestles). In recent years, detergent-based lysis has become very popular due to ease of use, low cost and efficient protocols.

Mammalian cells have a plasma membrane, a protein-lipid bilayer that forms a barrier separating cell contents from the extracellular environment. Lipids comprising the plasma membrane are amphipathic, having hydrophilic and hydrophobic moieties that associate spontaneously to form a closed bimolecular sheet. Membrane proteins are embedded in the lipid bilayer, held in place by one or more domains spanning the hydrophobic core. In addition, peripheral proteins bind the inner or outer surface of the bilayer through interactions with integral membrane proteins or with polar lipid head groups. The nature of the lipid and protein content varies with cell type. Clearly, the technique chosen for the disruption of cells, whether physical or detergent-based, must take into consideration the origin of the cells or tissues being examined and the inherent ease or difficulty in disrupting their outer layer(s). In addition, the method must be compatible with the amount of material to be processed and the intended downstream applications.

In particular embodiments, protein extraction also includes the pre-fractionation of cellular proteins originated from different compartments (such as extracellular proteins, membrane proteins, cytosolic proteins, nuclear proteins, mitochondrial proteins). Other pre-fractionation methods separate proteins on physical properties such as isoelectric point, charge and molecular weight.

According to a particular embodiment, the samples are pre-treated prior to labelling or cleavage, so as to denature the proteins for optimised access to reagents or proteases, using appropriate agents (e.g., guanidinium chloride, urea, acids (e.g. 0,1 % trifluoric acid), bases (e.g. 50 % pyridine) and ionic or non-ionic detergents).

Depending on the reagents used in the different embodiments of the methods of the present invention, it can be envisaged to reduce and modify the cysteine residues in proteins by thiol-reactive reagents. Widely used reagents for specifically modifying cysteine are iodoacetamide or vinylpyridine.

A first aspect of the present invention provides methods for the simultaneous analysis of protein cleavage events in two or more samples. The methods of the present invention comprise the following steps: modification of primary amines of proteins present in the samples, cleavage of the proteins and simultaneous labelling of the C-termini of the generated peptides, isolation ofN-terminal peptides, purification ofN-terminal peptides, and finally differential MS analysis of peptides.

Accordingly, the methods of the present invention comprise a step whereby the primary amine at the N-terminus of the protein(s) and the amines at the side chain of Lysine in the protein(s) present in the samples are modified. This is ensured by contacting the samples with a compound having an amine specific protein reactive group. Such reagent can bind to an amine in a reversible or irreversible way thereby making the amine group unavailable for amine-reactive reagents. This step is important as all primary amines in the protein(s) need to be modified before a selection ofN-terminal peptides on amine groups can be performed as explained in detail below. As a result of the modification of the free amine groups of the proteins in the samples, the samples will contain only peptides of which the N-termini are occupied, either as a result of the *in vitro* modification (described above) or due to their presence in the samples as blocked N-termini prior to the *in vitro* modification step.

In one embodiment the modification of primary amines is performed solely to remove these functional groups in the proteins in a sample. Suitable modification reagents in this context are amine-reactive reagents such as those described below.

Amine reactive reagents include carbamates (including methyl, ethyl, tert-butyl (e.g., Boc) and 9-fluorenylmethyl carbamates (e.g., Fmoc) amides), cyclic imide derivatives, N-Alkyl and N-Aryl amines, imine derivatives, and enamine derivatives. Other amine reactive agents are acetic anhydride, di-tert-butyl dicarbonate (i.e., Boc anhydride) or 9-fluorenylmethoxy carbonyl reagent (i.e., Fmoc reagent), which generates a 9-fluorenylniethoxy carbamate upon reaction with a reactive free amine. Examples of suitable Fmoc reagents include Fmoc-Cl, Fmoc-N₃, Fmoc-O-benzotriazol-1-yl), Fmoc-O-succinimidyl and Fmoc-OC₆F₅.

According to a particular embodiment, the step of modification of aminotermini in the methods of the present invention includes the selective modification of Lysine residues with a reagent prior to modification of the N-terminus. Lysine can be modified with O-methylisourea or O-methyl imidazole and its chemical derivatives (e.g., substituted O-methyl imidazole). These reagents selectively react with Lysine residues, without affecting free N-terminal amino groups, with the exception of polypeptides with N-terminal Glycine.

Some of these Lysine-modifying agents prevent enzymatic cleavage by Lysine-specific proteases, such as trypsin, which can be of interest to limit the cleavage of the protein in the enzymatic cleavage step.

In particular embodiments of the methods of the present invention, the step of modifying the primary amines present on the proteins in the samples is combined with a labelling step; According to this embodiment, the modification of the primary amines is exploited to ensure the incorporation of another label at the N-terminus of the peptides. Accordingly, in combination with the protease-induced labelling step, the labelling through the modification of the primary amines results in a double-labelling of (at least some of) the peptides in the samples.

Most particularly, the labels suitable for the labelling of peptides in the context of the present invention are isobaric labels (such as those described by Ross et al. ((2004) Mol. Cell. Proteomics 3, 1154-1169 and described in WO2004070352).

The concept of isobaric labelling is exemplified in Figure 1. Isobaric labelling reagents comprise a reporter group (RG), a balance group (BG) and a protein/peptide reactive group (PRG) as defined herein. In the embodiment illustrated in Figure 1, the complete isobaric labelling reagent consists of a reporter group based on *N*-methylpiperazine, a mass balance group which is a carbonyl, and a protein/peptide reactive group which is amine reactive group which is an NHS ester. While the mass of the reporter group is specific for each isobaric label within a set, the overall mass of the reporter group and the balance group of the different isobaric labels are kept constant. According to a particular embodiment this is ensured by using differential isotopic enrichment with ¹³C, ¹⁵N, and ¹⁸O atoms as indicated in Figure 1A. The isobaric labels depicted in Figure 1, which are commercially available, allow the introduction of four different labels at amine groups.

In view of the identical structure of the different isobaric reagents, the number and position of enriched centres in the ring has no effect on chromatographic or MS behaviour. Upon reaction of the amine specific reactive group of this labelling reagent with an amine functional group (N-terminal or amine group of lysine) in a peptide, the label becomes connected via an amide linkage to the peptide. These amide linkages fragment in a similar fashion to backbone peptide bonds when subjected to e.g. CID in MS/MS analysis. In the Example provided in Figure 1, the balance (carbonyl) moiety is lost (neutral loss) following fragmentation of the amide bond, while charge is retained by the reporter group fragment. The numbers in parentheses indicate the number of enriched centres in each section of the molecule.

Part B of Figure 3 illustrates the differences in the isotope distribution within reporter and balance groups used to arrive at four isobaric labelling reagents comprising four different reporter group masses. A mixture of identical peptides each labelled with a different member of the set of isobaric labelling reagents appears as a single, unresolved precursor ion in MS (identical m/z). Following CID, the four reporter group ions appear as distinct masses (114-117 Da). All other sequence-informative fragment ions (b-, y-, etc.) remain isobaric, and their individual ion current signals (signal intensities) are additive. This is also the case for those tryptic peptides that are labelled at both the N-terminus and Lysine side chains, and those peptides containing internal Lysine residues due to incomplete cleavage with trypsin.

The double labelling methods of the present invention thus allow, in MS/MS analysis, the determination of the relative concentration of the differentially labelled peptides, as it can be deduced from the relative intensities of the corresponding reporter ions. In contrast to ICAT and similar mass-difference labelling strategies, quantitation is thus performed at the MS/MS stage rather than in MS.

The labelling through primary amines of a peptide targets both the N-terminus and the amines of internal Lysines present in the peptides. According to one embodiment, the double labelling methods of the present invention do not comprise a modification step of the internal amine groups prior to the labelling steps and, accordingly isobaric labelling through the primary amines is entails that both the N-terminus and Lysine side chains of a protein are modified. N-terminal peptides comprising Lysine residues will accordingly carry more than one isobaric label.

Alternatively, the present invention envisages double labelling methods wherein, prior to the labelling steps, the samples are pre-treated such that Lysine is modified, e.g. a pre-treatment with a component such as O-methylisourea. As mentioned above O-methyliosurea does not react with N-terminal amines, with the exception of polypeptides with Glycine at the N-terminus. Hereafter the remaining free N-termini in the different samples are differentially labelled with an amine reactive isobaric label. The isobaric labelling reagent will not react with proteins with blocked (or previously modified) N-termini. A blocked N-terminus can be a naturally occurring blocked N-terminus or can be generated during sample processing (e.g. use of urea). The most frequent modification, N-acetylation, can be removed with enzymes (acylpeptide hydrolase) or by chemical methods (alcoholytic deacetylation). On the other hand, labelling only the free N-termini gives an additional reduction of the complexity of a sample, and can have advantageous properties. Thus depending on the type of assay and sample methods are envisaged either comprising the step of unblocking blocked N-termini and/or removal of the N-terminal modifications, or wherein N-terminal labelling is performed on the sample as such.

The methods of the present invention are characterized in that they comprise a step whereby the proteins in a sample are enzymatically cleaved and simultaneously isotopically labelled in one single step. Indeed, it has been determined that the enzymatic cleavage step traditionally performed in MS analysis and the labelling step can be combined to further rationalise the multiplex analysis.

According to a particular embodiment, the step of enzymatic cleavage is performed by treatment of the samples with trypsin, in the presence of either water (H₂¹⁶O) or H₂¹⁸O. Details on the trypsin mediated ¹⁸O incorporation are for example given in Heller et al. (2003) J. Am. Soc. Mass Spectrom. 14(7), 704-718. Upon enzymatic cleavage with trypsin, two O atoms are incorporated in the C-terminus of newly generated peptides. Accordingly, upon enzymatic cleavage with trypsin In the presence of H₂¹⁸O, two ¹⁸O atoms are incorporated in the C-terminus of newly generated peptides (see Figure 2). Of course, proteins which do not comprise a C-terminal Lysine or an Arginine will generate C-terminal peptides which do not have a ¹⁸O atom incorporated, such that not all c-terminal peptides in the sample will be isotopically labelled. This is however of no importance for the methods of the present invention as only the N-terminal peptides of the proteins are eventually analysed.

By differentially introducing either two ¹⁸O or two O by the combined cleavage and labelling step of the methods of the present invention, the resulting peptides become isotopically labelled with a mass difference of 4, without the attachment of additional groups to the peptide.

According to alternative embodiments, enzymes other than trypsin are used such as Lys-C, or Glu-C.

According to yet another embodiment, the step of cleaving of the proteins is performed using Peptidyl-Lys metalloendopeptidase (Lys-N). Cleavage with Lys-N results in the incorporation of only one ¹⁸O atom in the resulting peptide, which results in a mass difference of 2 between labelled and unlabelled species. This has the advantage that this enzyme does not generate a mixture of isotopically labelled peptides resulting from the incorporation of one or two ¹⁸O atoms into a peptide. Also Asp-N and chymotrypsin incorporate a single ¹⁸O upon cleavage (Schnolzer et al. (1996) Electrophoresis 17, 945-953).

It is noted that the enzyme-mediated isotopic labelling used in the methods of the present invention is specific for newly generated C-termini of cleaved peptides. The carboxylgroup of Asp and Glu is not modified. Thus contrary to prior art methods, there is no need to modify the functional groups of Asp and Glu in an additional method step prior to the isotopic labelling.

The methods of the present invention comprise the step of cleaving at least two different samples, each in the presence of either H₂O or H₂¹⁸O. Where the methods of the invention envisage a higher degree of multiplexing, e.g. by use of an additional labelling with isobaric labels as described above, the samples for labelling with either ¹⁸O or O will be selected such that a unique combinations of the isobaric labels with ¹⁸O or O are provided on the peptides in each sample, to allow differentiation of the peptides originating from proteins in the different samples.

This is illustrated in Figure 4. Using four different (commercially available) iTRAQ labels, two sets of four samples can be labelled with the individual iTRAQ labels. Each set of four samples can be pooled prior to the cleavage, whereafter one set of four samples is labelled with ¹⁸O during the cleavage, while the other is cleaved in the presence of water. In this way a differential labelling of 8 different samples is performed, which can be analysed as one pooled sample. Recently the set of commercially available iTRAQ labelling reagents has increased to 8, allowing an even larger multiplexity.

As a result of the N-terminal blocking step and the combined cleavage and labelling step of the present invention, all N-terminal peptides of the proteins present in the sample will comprise either O or the ¹⁸O isotope. In addition all internal and C-terminal peptides have a free N-terminus while all N-terminal peptides have a modified N-terminus, either because it was blocked as such in the sample or as a result of the modification (and optionally labelling) step.

The methods of the present invention further comprise an isolation step, wherein the internal and C-terminal peptides of the proteins in the samples are removed from the N-terminal peptides of the cleaved proteins. Although different samples can be treated separately, in general all samples of an assay are pooled prior to the step of isolating the N-terminal peptides.

According to one embodiment the internal and C-terminal peptides, comprising a free N-terminus are bound to or reacted with a matrix that is specific for primary amines. Examples hereof are Ni²⁺-chelated NTA (Nitrilotriacetic acid) materials provided on magnetic beads, sepharose or agarose resins, etc.

According to another embodiment the N-terminus of the internal and C-terminal peptides is reacted with an affinity tag. Examples of affinity tags include:
- d-biotin or structurally modified biotin-based reagents, including d-iminobiotin,
- 1,2-diols, such as 1,2-dihydroxyethane (HO-CH₂ -CH₂ -OH), and other 1,2-dihydroxyalkanes including those of cyclic alkanes, e.g., 1,2-dihydroxycyclohexane which bind to an alkyl or aryl boronic acid or boronic acid esters, such as phenyl-B(OH)₂ or hexyl-B(OEthyl)₂ (e.g. attached via an alkyl or aryl group to a support such as agarose)
- maltose which binds to Maltose Binding Protein; or other sugar/Sugar Binding Protein pairs, or more generally to any ligand/Ligand Binding Protein pair which obeys to the above mentioned criteria of affinity tags.
- a hapten, such as dinitrophenyl group, which binds to the corresponding anti-hapten antibody such as anti-dinitrophenyl-IgG;
- a ligand which binds to a transition metal, for example, an oligomeric histidine (so called 6His-tag) will bind to Ni(II), the transition metal CR is in particular embodiments used in the form of a resin-bound chelated transition metal, such as nitrilotriacetic acid-chelated Ni(II) or iminodiacetic acid-chelated Ni(II);
- glutathione which binds to glutathione-S-transferase.

In particular embodiments of the methods of the present invention, the internal and C-terminal peptides are discarded and are not used for further analysis. Thus, in these embodiments, there is no need for a reversible binding of these peptides with the matrix, or for affinity tags which can be released from the affinity matrix with a displacing ligand, or for affinity tags with a cleavable linker between the tag and the peptide. If however, the recovery of internal and C-terminal peptides is considered, reversible binding, displacement ligands or cleavable affinity tags are optionally used.

In particular embodiments the affinity tag used for the removal of internal and C-terminal peptides from the peptide sample(s) is biotin. Reagents for binding biotin to amine groups are commercially available and include for example succinimidyl D-biotin, 6-((biotinoyl)amino)hexanoic acid, succinimidyl ester and 6-((6-((biotinoyl)amino)hexanoyl)amino) hexanoic acid, succinimidyl ester. Biotin affinity tagged peptides are bound via conventional avidin-, or streptavidin affinity chromatography on column or on beads. Instructions for use can be found e.g. in the technical data sheets from Pierce (Rockville, IL).

In the above described separation methods, the N-terminal peptides will not bind and are recuperated as the non-binding fraction, and are thus indirectly selectively isolated for further analysis.

The methods of the invention provide for simultaneous analysis of differentially labelled samples to improve accuracy of the comparison between these samples. Accordingly, the methods of the invention comprise the step of pooling the different samples for analysis. As indicated above, the differentially labelled and cleaved protein samples can be pooled prior to the selective isolation of the N-terminal peptides. Alternatively, the selective isolation of N-terminal peptides is performed on individual (or partly pooled) samples and the different fractions of N-terminal peptides are pooled at this stage.

The methods of the invention further comprise one or more separation steps, which are typically performed after the isolation of the N-terminal peptides or (where appropriate) the pooling of the different N-terminal peptide fractions. According to the present invention, the nature of the differential labelling, both with regard to isotopic O labelling and the optional isobaric labelling is such that the chemical structure of the labels is the same. Thus, the chemical structure of the labels present on each of the differentially labelled samples is the same, such that it will not generate a significant difference in properties in (multi-dimensional) chromatography techniques, between identical peptides that are differently single or double-labelled. Accordingly, labelled peptides with the same amino acid sequence will behave identically and will remain in the same fraction.

Suitable separation techniques, which allow the separation of a complex peptide sample into multiple fractions are known to the skilled person and include, but are not limited to isoelectric focusing, ion exchange chromatography, reversed-phase HPLC, affinity chromatography, ... etc. Techniques such as SDS PAGE, 2-dimensional gel electrophoresis, size-exclusion chromatography are less suited for the N-terminal peptides of generally limited length, which were isolated in the previous method step.

For peptide samples obtained from e.g. proteolytic digestions, 2D LC approaches are more suitable for separation, and also the automation and throughput is significantly better. Several technologies to separate peptide digests by liquid chromatography have been described, including, reversed-phase (RP)-HPLC, and multidimensional liquid chromatography. Also capillary electrophoresis (CE) is a method suitable for the separation of peptides.

2D-LC generally uses ion-exchange columns (usually, strong cation exchange, SCX) on-line coupled with a reversed phase column, operated in a series of cycles. In each cycle the salt concentration is increased in the ion-exchange column, in order to elute peptides according to their ionic charge into the reversed phase system. Herein the peptides are separated on hydrophobicity by e.g. gradient with CH₃CN.

Many parameters influence the resolution power and subsequently the number of proteins that can be displayed by LC-MS. Usually, the 'on-line' configuration between the first-dimension separation technique (SCX) and the second-dimension RP-HPLC separation approach is set up for sample fractionation. Ion exchange chromatography can be performed by stepwise elution with increasing salt concentration or by a gradient of salt. Typically, SCX is performed in the presence of, e.g. up to 30% acetonitrile, to minimize hydrophobic interactions during SCX chromatography. Prior to Reversed Phase chromatography on e.g. a C18 column, organic solvents such as acetontrile are removed, or strongly reduced by e.g. evaporation.

The methods of the invention further comprise the step of identifying peptides for which differential processing has occurred in the different samples. This identification step is ensured by detecting the differential mass of the peptides in the samples (in MS or MS/MS) and determining the sequence thereof. The sequence of the peptides can be reconstituted based on the information generated in MS/MS analysis of the identified peptides. Accordingly, the methods of the present invention comprise the step of analysing the peptides or peptide fractions comprising the double-labelled peptides in MS and MS/MS.

The following describes how the information obtained in MS and MS/MS can be used to gain information on differential proteolytic processing of proteins in samples.

As detailed herein, the spectrum generated on a mass spectrometer of an N-terminal peptide which has been isolated from a pool of differentially isotopically labelled samples, contains in principle a pair of peaks with a characteristic mass difference of 2 or 4 (depending on the enzyme used), as a result of the isotopic labelling (¹⁶O versus ¹⁸O) of the two samples or two sets of samples. Where only two samples are involved, due to proteolytic processing or differential expression (as a direct or indirect consequence of proteolytic processing) of the protein corresponding to this N-terminal peptide in the two samples analysed, only one peak can be present. This is detailed below.

The absence of one of the isotopes of a peptide in a MS spectrum can have different reasons.

First, it can be caused by the differential *in vivo* processing of the protein in the two samples. Where differential processing occurs, the peptides which generate peaks on MS will be different depending on the nature of the processing. Table 1 exemplifies different options for a theoretical peptide. The resulting N-terminal peptides of an unprocessed control protein (see (1) in Table 1 below), a protein that is processed in the N-terminal peptide (i.e. N-terminally of the cleaving with trypsin, see (2) in Table 1), a protein that is processed at an amino acid which is also the cleaving site for trypsin (see (3) in Table 1) and a protein that is processed at a location which falls within in an internal peptide upon cleavage with trypsin (see (4) in Table 1) are provided. In this Example, the unprocessed control protein is cleaved in the presence of water, while experimental samples comprising either (2), (3) or (4) are cleaved with trypsin in the presence of H₂¹⁸O.

Table 1: Identification of N-terminal peptides resulting from a protein which is not processed (1) or processed in different locations in the protein (2, 3, 4). The location of processing is defined relative to the peptides generated by trypsin cleavage, i.e. within the N-terminal peptide (A), within the internal peptides (B) or (C) or within the C-terminal peptide (D). T₁, T₂ and T₃ correspond to a tryptic cleavage point (Lys or Arg) separating these peptides. Y and Z are hypothetical sites for proteolytic *in vivo* processing within the trypsin peptides. #: N-terminal modification. ¹⁶O and ¹⁸O: isotopic labelling with respectively normal water and H₂¹⁸O. Where processing occurs at amino acids Y or Z; the C-terminus of the resulting peptide is not generated as a result of trypsin cleavage and ¹⁸O is not incorporated. Similarly, the C-terminus of the protein is not generated by trypsin cleavage and thus does not incorporate the ¹⁸O isotope. As a result of processing, peptides A and B are split up in, respectively A' and A", and B' and B".A: list of peptides occurring under different conditions; B: peaks generated on MS upon pooling of different samples corresponding to the conditions of (A), each column representing a region of peaks corresponding to isotopic peptides.

| A. | | | |
|---|---|---|---|
| Conditions | Corresponding protein/peptides | | |
| (1) modified non- | #NH₂--(A)Y-T₁--(B)Z--T₂--(C)--T₃-- (D)--COOH | | |
| processed protein | | | |
| cleaved and labelled | #NH₂--(A)Y-T₁¹⁶O | | |
| peptides (¹⁶O) | NH₂--(B)Z--T₂¹⁶O | | |
| | NH₂--(C)--T₃¹⁶O | | |
| | NH₂--(D)-COOH | | |
| isolated N-terminal | #NH₂--(A)Y-T₁¹⁶O | | |
| peptide(s) | | | |
| (2) modified protein | #NH₂-(A')Y | | |
| processed at Y | #NH₂-A"- T₁--(B)Z-- T₂--(C)-T₃-- (D)--COOH | | |
| cleaved and labelled | #NH₂-(A')Y | | |
| protein (¹⁸O) | #NH₂-(A")-T₁¹⁸O | | |
| | NH₂--(B)Z--T₂¹⁸O | | |
| | NH₂--(C)--T₃¹⁸O | | |
| | NH₂-- (D)-COOH | | |
| isolated N-terminal | #NH₂-(A')Y | | |
| peptide(s) | #NH₂-(A")-T₁¹⁸O | | |
| (3) modified protein | #NH₂--(A)Y-T₁ | | |
| processed at T₁ | #NH₂--(B)Z--T₂--(C)--T₃-- (D)--COOH | | |
| cleaved and labelled | #NH₂--(A)Y-T₁¹⁸O | | |
| peptides (¹⁸O) | #NH₂--(B)Z--T₂¹⁸O | | |
| | NH₂--(C)-- T₃¹⁸O | | |
| | NH₂--(D)-COOH | | |
| isolated N-terminal | #NH₂--(A)Y-T₁ ¹⁸O | | |
| peptide(s) | #NH₂--(B)Z--T₂¹⁸O | | |
| (4) modified protein | #NH₂--(A)Y-T₁--(B')Z | | |
| processed at Z | #NH₂-B"-T₂--(C)--T₃-- (D)--COOH | | |
| cleaved and labelled | #NH₂--(A)Y-T₁¹⁸O | | |
| peptides (¹⁸O) | NH₂--(B')Z | | |
| | #NH₂-B"-T₂¹⁸O | | |
| | NH₂--(C)--T₃¹⁸O | | |
| | NH₂--(D)-COOH | | |
| isolated N-terminal peptide(s) | #NH₂--(A)Y-T₁¹⁸O | | |
| | #NH₂-B"-T₂¹⁸O | | |

| B | | | |
|---|---|---|---|
| pooled samples | detected peptides on MS (in different regions of the spectrum) | | |
| 1+2 | #NH₂--(A)Y--T₁¹⁶O | #NH₂-(A')Y | #NH₂-(A")-T₁¹⁸O |
| | | | |
| 1+3 | #NH₂--(A)Y--T₁¹⁶O | #NH₂--(B)Z--T₂¹⁸O | |
| | #NH₂--(A)Y--T₁¹⁸O | | |
| 1+4 | #NH₂--(A)Y--T₁¹⁶O | #NH₂-(B")-T₂¹⁸O | |
| | #NH₂--(A)Y--T₁¹⁸O | | |

The different situations illustrated in Table 1 are commented briefly below.

In the first situation a protein is not processed in one sample (see (1) in Table 1) but in the other sample (see (2) in Table 1) is processed *in vivo* at a position (Y) N-terminally of the first cleavable amino acid T₁ for the cleavage/labelling step. The single MS signals which are generated after pooling and chromatography can correspond to the N-terminal peptide (A) of the intact protein, or to either N-terminus (A') or the newly generated N-terminus (A"), resulting from the processing within (A). The peptide A' will not be isotopically labelled since its C-terminus is generated by processing and not by cleavage in the presence of H₂¹⁸O (thereby assuming that Y is not a cleaving site for trypsin).

It is noted that the chance that after processing as illustrated in (2), the peptide (A') resulting from processing of (A) is in fact still present is low, most particularly when the processing is the result of an aminopeptidase or a dipeptidase or another enzyme which N-terminally cleaves off a peptide of 5 or 10 amino acids or less.

In the second situation a protein is not processed in one sample (1) and is in another sample (see (3) in Table 1) processed at position T₁ which is also a cleavable amino acid for the cleavage/labelling step. In this case also the processed peptide A will be isotopically labelled and peptides (A) will appear in its two isotopic forms in the MS spectrum after pooling and chromatography. The single peak which is noticed in the MS spectrum corresponds to the novel N-terminal peptide B of the processed protein.

In the third situation a protein is not processed in one sample (1) and is in another sample (see (4) in Table 1 and also figure 3) processed in an internal peptide (B) at position Z. The processed part of peptide (B), the peptide (B') behaves as a C-terminal peptides is discarded during the step of isolating N-terminal peptides

The single MS signal, which will appear after pooling and chromatography corresponds to N-terminal peptide (B") of the processed protein.

Table 1 illustrates *in vivo* processing in an internal peptide near the N-terminus of a protein. The processing can however in many proteins also occur further away from the N-terminus. For example, the processing of Van Willebrand factor by Furin occurs at position 763 in a protein of 2813 amino acids.

In theory, each protein which is differently processed between two samples should nevertheless reveal in MS an N-terminal peptide, either from the unprocessed protein, from the novel N-terminus generated as a result of the processing of the protein. Sequence determination of the novel N-terminal peptide will also reveal the site of processing in the protein. The amino acid sequence around the cleavage site can comprise a motif which is recognised by certain proteases. In this way information can be obtained about the (type of) protease that caused the cleavage.

Alternatively, proteolytic processing can lead to the degradation of the processed protein such that no N-terminal peptide is recovered at all for the processed protein in this sample. A single peak of the N-terminus of the non-processed protein will appear in MS. In this situation the analysis indicates a difference in processing but does not reveal at which position in the protein the processing occurs.

Yet a third possibility is that the absence of the N-terminal peptide in one of the samples is caused by downstream effects of the processing. For example, inefficient processing of protein can lead to a deficient signalling in a pathway and subsequently to lowered or absent of transcription and translation of genes which are controlled by that pathway.

When unknown proteins are analysed it can not be excluded that the presence of only one N-terminal peptide is caused by mutations within the N-terminal peptide within one of the samples or by alternative splicing which results in the use of alternative ATG (downstream or upstream of the normal initiator gene). In these cases different N-terminal peptides are present in the two samples, which will elute as different fractions during chromatography and thus will not be analysed in one fraction by MS.

Alternatively, an N-terminal peptide of a protein of one sample can be present in a lower or higher amount compared to the N-terminal peptide of another (or the control) sample. In this situation, the difference can be explained by differences in stability due to different processing, differences in shedding, or differences in gene expression due to downstream effect of proteolytic processing as explained above.

The use of only two samples wherein one is labelled with ¹⁸O has certain advantages. The pool of peptides which are subjected to purification contains the N-terminal peptides of all proteins in the sample. The different proteins and peptides are then separated into fractions prior to MS. However, of these proteins a large number are not processed or are processed in an identical way in both samples. All the N-terminal peptides of these (unprocessed) proteins will appear on MS as two peaks of about the same intensity and can be neglected for further analysis. However where the object of the method is to verify a known processing of a certain protein, one can analyse specifically those peaks which correspond to the mass of the predicted N-terminal peptide of an intact of processed protein. Only those peptides fractions for which the two peaks (corresponding to the differentially isotopically labelled peptides) have a significant difference in intensity on MS (ratio between the peaks of the isotopically labelled peptides is below 0,5 or above 1,5.), or where one of the two peaks is absent (or practically absent, i.e. the ratio between the peaks of the isotopically labelled peptides is below 0,1 or above 10 or even below 0,05 or above 20), are indicative of a differential processing of the peptide, and thus are of interest for further analysis. Accordingly, particular embodiments of the present invention encompass a method for simultaneous analysis of two samples wherein, after cleavage and concomitant labelling with an isotopic label, the samples are pooled, N-terminal peptides are isolated and separated and analysed by MS, and the selection of the relevant peptides in MS consists of identifying those peaks for which the ratio between the peaks of the isotopically labelled peptides is below 0,5 or above 1,5.

When double labelling is performed (i.e. combination of isobaric labels and isotopic O labels), so as to allow the combined analysis of more than two samples, MS analysis will only differentiate the two groups of isotopically labelled proteins, each of these groups comprising the proteins from samples differentially labelled with an isobaric label. Accordingly, the chance that only one peak corresponding to one isotope is observed on MS will be smaller (as it would require that in all of the samples labelled with that isotope the processing of that protein is affected). Nevertheless, differences in the relative intensity between the two peaks generated in MS are indicative of the fact that one of the peptides of one isotopic form is absent or present in a lower concentration. On the other hand, in the MS analysis of a larger number of pooled samples, the presence of two isotopic peptides of equal intensity does not mean that no processing of the relevant protein has occurred in any of the samples, as the MS peaks only provide the cumulative intensity for the different peptides labelled with the same isotope. Individual differences can be compensated by other samples or can fail to be noticed if a sample for each of the isotopes is similarly affected. Such phenomenon can be avoided to some extent by the design of the experiment, more particularly the choice of isotope label for each sample. For example when a protease is known to be linked with cancer, one isotope is used to label a sample from an affected patient and a cell culture of healthy cells wherein a construct is transfected that over-expresses the protease. The other isotope is used to label a tissue from a healthy person and a cell culture of healthy cells wherein a construct is transfected with an inactive from of the protease as a control. By this design differences in MS are more likely to be observed when different proteolytic processing occurred.

Where double-labelling is performed, the different peaks on MS corresponding to the different isotopically labelled samples are each further analysed on MS/MS to further differentiate between the differential isobarically labelled peptides. In MS/MS a spectrum will be generated of the different reporter groups generated by CID. Other suitable methods to fragment peptides include CAD (collisionally activated dissociation), ETD (electron transfer dissociation), ECD (electron capture dissociation), IRMPD (infrared multiphoton dissociation) and BIRD (blackbody infrared radiative dissociation

The absence or the difference in concentration of a peak corresponding to the reporter group of a certain peptide can also be indicative of different processing, different stability and different downstream effects in the sample from which the peptide originates, as explained above.

In this regard it is noted that an N-terminal peptide, which is derived from a blocked N-terminus will not be labelled at its N-terminus by the isobaric label, and will only carry isotopic label upon enzyme mediated ¹⁸O incorporation. Differentially isotopic labelled peptides are separated during MS. However at the subsequent MS/MS no reporter groups will be identified.

Analysis of changes in cleavage patterns of proteins are of value in linking altered proteolysis with disease. For example, in a clinical assay, any specific type of proteolytic cleavage, e.g., by an aspartic, cysteine, metallo, serine/threonine or other type of protease, can be determined as described herein by identifying the N-terminal peptides by mass spectrometry, and any changes in the observed cleavage patterns over time can be followed.

The methods of the present invention are suitable to identify biomarkers, to monitor the therapeutic response to specific protease inhibitor, to select and screen drug candidates in preclinical studies, to select patients and their response in clinical drug development, to design peptide inhibitors for specific proteases, to identify novel proteases, and to gain knowledge about the mechanism of disease aetiology. The methods can also be used to detect aberrant protein expression or function due to genetic mutations that may result in proteolytic degradation and subsequent peptide generation.

Alternatively, the methods of the present invention are used to explore the set of substrates for a selected protease, e.g., a metalloprotease, in a sample. Herein, a plasma or cell extract is incubated with a protease and the site(s) of hydrolysis is/are determined. This information allows to look for the same patterns in a specific disease and to use the observation of an associated panel of peptides in the sample to show that the protease is upregulated in that disease. Furthermore, information concerning the peptides of the degradome can be used to look at the pathology of a diseased sample by determining the cleavage patterns either in the tissue or in a fluid and then using that information to identify the protease. Alternatively, the methods of the invention are used to identify a set of proteases up or down-regulated in a specific disease or condition.

The present invention provides tools and methods for the simultaneous identification (by MS/MS) and/or quantitation (by MS or MS/MS) of N-terminal peptides in different samples. More particularly, the methods of the present invention relate to the identification of proteolytically processed proteins from different samples in MS and MS/MS using differential isotopic labelling an optionally additional isobaric labelling. Accordingly, the devices for performing the methods of the present invention comprise one or more mass spectrometric instruments.

Mass measurements by spectrometry are performed by the ionisation of analytes into the gas phase. A typical mass spectrometric instrument consists of 3 components, an ion source generate ions from the molecules of interest, a mass analyser, which determines the mass-to-charge ratio (m/z) of the ionised molecules, and a detector that registers and counts the number of ions for each individual m/z value. Each feature in an MS spectrum is defined by two values, m/z and a measure on the number of ions, which reached the detector of the instrument.

The ionisation of proteins or peptides for mass analysis in a spectrometer is usually performed by Electro-Spray Ionisation (ESI) or Matrix-Assisted Laser Desorption/Ionisation (MALDI).

During the ESI process analytes are directly ionized out of solution and ESI is therefore often directly coupled to liquid- chromatographic separation tools (e.g., reversed phase HPLC), MALDI vaporises via laser pulses dry samples mixed with small organic molecules like cinnamic acid that absorb the laser energy to make the process more effective by the addition of small organic molecules. MALDI-MS is normally used to analyse relatively simple peptide mixtures, whereas integrated liquid-chromatography MS systems (LC-MS) are preferred for the analysis of complex samples.

The mass analyser is a key component of the mass spectrometer; important parameters are sensitivity, resolution, and mass accuracy. There are five basic types of mass analysers currently used in proteomics. These include the ion trap, time-of-flight (TOF), quadrupole, Orbitrap, and Fourier transform ion cyclotron (FTICR-MS) analysers. Tandem MS or MS/MS can be performed in time (ion trap) and in place (with all hybrid instruments such as e.g. LTQ-FTICR, LTQ-Orbitrap, Q-TOF, TOF-TOF, triple quad and hybrid triple quadrupole/linear ion trap (QTRAP))

The methods of the invention further comprise one or more peptide separation steps. Accordingly devices suitable for performing the methods of the present invention optionally contain or are connected to one or more suitable separation instruments, such as electrophoresis instruments, chromatography instruments, such as, but not limited to capillary electrophoresis (CE) instruments, reverse-phase (RP)-HPLC instruments, and/or 2-dimensional liquid chromatography instruments,... etc.

According to one embodiment, the devices of the present invention (Figure 5) are suitable for analysis of two protein samples using isotopic labelling (100') and comprise two sample sources (101), protein modification unit (103') with a source of modifying reagent (104'), a cleavage and labelling unit (105) with corresponding ¹⁸O and ¹⁶O sources (107), an N-terminal peptide isolation unit (106) a separation unit (108), a mass spectrometer unit (109) and a control circuitry and data analysis unit (110) coupled to a read out system (111). In particular embodiments separation unit (108) comprises two consecutively linked separation systems (1108) and (2108), wherein e.g. (1108) is a cation exchange chromatography system and separation system (2108) is typically a HPLC reversed phase system. Mass spectrometer element (109) can be an MS spectrometer but is typically an MS/MS spectrometer which separates isotopic forms and wherein *de novo* peptide sequencing can be performed. MS/MS analysis can be done using 2 fundamentally different instruments. In the first type of instrument, the ion trap in which MS/MS analysis is done in the same iontrap where MS is performed, but MS/MS is done in time (trap is filled, all ions are ejected except ion(s) of interest and CID is performed and the fragment ions are scanned. The second type of instruments, hybrid instruments (triple quad, q-tof, ltq-ftms, ltq-orbitrap), separate MS/MS in place. e.g. parent selection is done in the first mass analyzer and fragments are scanned in the second mass analyzer. The devices according to this embodiment can further comprise a number of optional elements such as a sample preparation unit (102) wherein e.g. sample lysis and immunodepletion takes place. Optionally, an additional modification unit is included with a corresponding modification reagent source, which allows modification of the amine function internal Lysines as described herein. This modification unit is placed such that modification of the samples takes place prior to protein cleavage.

A further embodiment of the invention devices (100) are provided for multiplex analysis of protein samples using double labelling (Figure 5) comprising at least two sample sources (101), a first labelling unit (103) with corresponding first label sources (104), a cleavage unit and second labelling unit (105), with corresponding second label sources (107) an N-terminal peptide isolation unit (106) a separation unit (108), a mass spectrometer unit (109) and a control circuitry and data analysis unit (110) coupled to a read out system (111). In particular embodiments separation unit (108) comprises two consecutively linked separation systems (1108) and (2108), wherein e.g. (1108) is a cation exchange chromatography system and separation system (2108) is typically a HPLC reversed phase system. The mass spectrometer element (109) is an MS/MS spectrometer as described above, wherein additionally in the double labelling methods of the present invention, the reporter groups of the isobaric labels generated upon CID are differentially detected. This device can further comprise a number of optional elements such as a sample preparation unit (102) wherein e.g. sample lysis and immunodepletion takes place. Similar to the device described above, an additional modfication unit, for the modification of the amine function of Lysines is included.

Another aspect of the invention thus provides combinations of reagents and kits comprising such reagents, suitable for carrying out the methods of the present invention.

According to one embodiment, the reagents comprise a set of two or more isobaric labels and H₂¹⁸O.

Optionally, kits are provided which include both suitable reagents and means, which are optionally disposable for performing the steps of isolating the N-terminal peptides. The latter means optionally comprise disposable solid phase chromatography which allow efficient removal of C-terminal and internal peptides from the individual or pooled samples.

Other arrangements of the methods, systems, device and kits embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1: Isotopic labelling of N-terminal peptides.

Two protein samples (1 and 2) are modified on the N-terminus and on lysine with acetic acid anhydride. One sample is digested with trypsin in the presence of normal water (16). The other sample is digested with trypsin in the presence of water with a heavy ¹⁸O isotope (18).

The peptides of both samples are pooled. The newly generated N-termini on the internal and C-terminal peptides are modified with biotin and isolated by avidin affinity chromatography.

The N-terminal peptides are subjected to ion exchange chromatography and reverse phase chromatography. Each peptide fraction is analysed by MS wherein the peptides with ¹⁶O isotope and ¹⁸O isotope are separated. The ratio of both peaks is calculated. The sequence of the peptides is determined by MS/MS. The sequences are compared with sequence database to determine eventual proteolytic processing.

### Example 2 : Isobaric/isotopic double labelling of N-terminal peptides.

8 samples (1 to 8) are labelled with 4 different isobaric labels (A to D) (as depicted in Figure 4) on the N-terminus of the proteins in the sample. Labelled samples 1 to 4 and 5 to 6 are pooled. One pool (samples 1 to 4) is digested with trypsin in the presence of normal water (16). The other pool (samples 5 to 6) are digested with trypsin in the presence of water with a heavy ¹⁸O isotope (18).

The peptides are modified with biotin and internal and C-terminal peptides are isolated by avidin affinity chromatography

The N-terminal peptides of all double-labelled samples are pooled and subjected to ion exchange chromatography and reverse phase chromatography. Each peptide fraction is analysed by MS wherein the peptides with ¹⁶O isotope and ¹⁸O isotope are separated. Each isotope is subsequently analysed by MS/MS wherein the different isobaric forms release the reporter group and wherein the sequence of the peptides is determined. The relative concentration of different peptides is calculated from the individual reporter groups and isotopes.

## Claims

1. An *in vitro* method for investigation differences in proteolytic processing between two or more different samples comprising the steps of:
a) modifying the amine of the N-terminus and of Lysine of the proteins in said samples,
b) cleaving the modified proteins into peptides and simultaneously labelling each of the samples with either O or ¹⁸O,
c) isolating N-terminal peptides,
d) pooling of the labelled samples after step (b) or of the isolated N-terminal peptides of step (c),
e) subjecting the N-terminal peptides to MS,
f) selecting relevant peptide fractions for further analysis, and
g) identifying peptides which are generated by proteolytic processing.

2. The method of claim 1, which further comprises, after step (d), the step of
- subjecting the isolated N-terminal peptides to a peptide separation step.

3. The method according to claim 1 wherein in step (a) the modification is performed on each sample with a different isobaric labelling reagent comprising an amine reactive group.

4. The method according to claim 1, wherein the cleavage in step (b) is performed with trypsin.

5. The method according to claim 1, wherein the isolation of N-terminal peptides is performed by covalently linking an affinity tag to the N-terminus of the internal and C-terminal peptides, and removing the internal and C-terminal peptides from the samples by affinity chromatography.

6. The method according to claim 1 wherein step (g) comprises analysing the identified protein samples on MS/MS.

7. The method according to claim 1 wherein, when two samples are used, the selection step in step (f) consists of identifying those peaks for which the ratio between the peaks of the isotopically labelled peptides is below 0,5 or above 1,5.

8. The method according to claim 1 wherein, when two samples are used, the selection step in step (f) consists of identifying those peaks for which the ratio between the peaks of the isotopically labelled peptides is below 0,1 or above 10.

9. Use of the method of claim 1 for determining proteolytic cleavage sites.

10. Use of the method of claim 1 for determining downstream effects of proteolytic processing.

11. The method according to claim 1 wherein one or more of the protein samples are body samples from tumour patients.

12. A kit of reagents comprising a set of two or more isobaric labelling reagents and H₂¹⁸O.

13. The kit according to claim 12, further comprising means for isolating polypeptides with a free N-terminus.

14. A device (100') for analysis of two protein samples using isotopic labelling comprising two sample sources (101), a protein modification unit (103') with a source of modifying reagent (104'), a labelling and protein cleavage unit (105) and corresponding label sources (107), a N-terminal peptide isolation unit (106), a separation unit (108), a mass spectrometer unit (109) and a data analysis unit (110).

15. A device (100) for multiplex analysis of protein samples using double labelling comprising at least two sample sources (101), a labelling unit (103) with at least two sources of labelling reagent (104), a labelling and protein cleavage unit (105) and corresponding label sources (107), a N-terminal peptide isolation unit (106) a separation unit (108), a mass spectrometer unit (109) and a data analysis unit (110).

16. The device according to claim 14 or 15, further comprising a sample preparation unit (102).
